**Europäisches Patentamt**

**European Patent Office**

**Office européen des brevets**

(19)

(11) Veröffentlichungsnummer: **0 097 193 B1**

(12)

# EUROPÄISCHE PATENTSCHRIFT

(45) Veröffentlichungstag der Patentschrift:
**26.08.87**

(51) Int. Cl.⁴: **A 61 N 5/06**

(21) Anmeldenummer: **83900034.6**

(22) Anmeldetag: **23.12.82**

(86) Internationale Anmeldenummer:
**PCT/EP 82/00273**

(87) Internationale Veröffentlichungsnummer:
**WO 83/02233 (07.07.83** Gazette 83/16)

(54) **UV-BESTRAHLUNGSGERÄT ZUR PHOTOTHERAPIE VON DERMATOSEN, INSBESONDERE VON PSORIASIS.**

(30) Priorität: **24.12.81 DE 3151494**

(43) Veröffentlichungstag der Anmeldung:
**04.01.84 Patentblatt 84/1**

(45) Bekanntmachung des Hinweises auf die Patenterteilung:
**26.08.87 Patentblatt 87/35**

(84) Benannte Vertragsstaaten:
**AT BE CH DE FR GB LI NL SE**

(56) Entgegenhaltungen:
**EP - A - 0 052 765**
**DE - A - 2 531 876**
**DE - A - 2 707 920**
**DE - A - 2 803 446**
**FR - A - 2 347 943**
**FR - A - 2 389 229**
**GB - A - 813 118**
**US - A - 2 811 671**
**US - A - 4 298 005**

**Applied Optics, volume 19 N0 2, January 1980, American Institute of Physics New York (US) S E. Whitcomp et al.:"Low-pass interference filters for submilimeter astronomy" ;pages 197 to 198;**

(73) Patentinhaber: **Mutzhas, Maximilian F., Prof. Dr., Sonnenstrasse 17, D-8000 München 2 (DE)**

(72) Erfinder: **Mutzhas, Maximilian F., Prof. Dr., Sonnenstrasse 17, D-8000 München 2 (DE)**

(74) Vertreter: **Tetzner, Volkmar, Dr.-Ing. Dr. Jur., Van-Gogh-Strasse 3, D-8000 München 71 (DE)**

## Beschreibung

Die Erfindung betrifft ein UV-Bestrahlungsgerät zur Phototherapie von Dermatosen, insbesondere von Psoriasis, gemäss dem Oberbegriff des Anspruchs 1.

Etwa 2% der Bevölkerung leiden an Psoriasis, einer durch einen Gendefekt hervorgerufenen Schuppenflechte. Durch eine stark gesteigerte Zellteilungsrate (Zellproliferation) in der Basalschicht der Epidermis entstehen auf der Hautoberfläche die schuppigen Psoriasisherde, die zu starken physischen und psychischen Beeinträchtigen führen.

Ziel der Psoriasis-Therapie ist es, die Herde zurückzubilden, um die Haut schuppenfrei zu bekommen und sie in diesem Zustand zu erhalten. Zur Therapie werden im Augenblick die Behandlungsformen der Chemotherapie, der Photochemotherapie und der Phototherapie eingesetzt.

Bei der Chemotherapie werden Medikamente oral verabreicht oder auf die Haut aufgetragen. Dabei sind meist nicht nur erhebliche Nebenwirkungen, sondern teilweise auch Schmerzen in Kauf zu nehmen. Nachteilig ist ferner der Umstand, dass eine Chemotherapie häufig nur stationär in Kliniken durchgeführt werden kann.

Die Photochemotherapie verwendet Medikamente in Verbindung mit einer UV-Bestrahlung. Bei der seit langem angewendeten Goeckermann-Therapie wird Teer auf die Haut aufgetragen und anschliessend eine UV-Bestrahlung durchgeführt. Um einen Therapie-Erfolg zu erreichen, muss hierbei solange bestrahlt werden, bis die Haut ein Erythem (Sonnenbrand) aufweist.

Eine moderne Art der Photochemotherapie ist unter der Bezeichnung PUVA bekannt. Hierbei wird ein photosensibilisierendes Medikament (Psoralen) oral verabreicht oder auf die Haut aufgetragen. Anschliessend erfolgt eine Bestrahlung mit UV-A im Wellenlängenbereich zwischen 310 und 440 nm. Das photosensibilisierende Medikament dient hierbei dazu, die Haut für die langwellige UV-A-Strahlung empfindlicher zu machen.

Als UV-Strahlungsquellen finden bei diesem bekannten Verfahren Geräte Verwendung, die entweder UV emittierende Leuchtstofflampen (UV-A-Niederdruckstrahler) enthalten oder Quecksilber-Hochdruckstrahler, Xenonstrahler oder mit Metallhalogenen dotierte Quecksilber-Hochdruckstrahler. Die unterhalb 315 nm liegende kurzwellige Strahlung wird bei diesen Geräten meist durch Filter unterdrückt.

Um einen Therapie-Erfolg zu erreichen, muss auch bei diesem bekannten Therapie-Verfahren ein Erythem erzeugt werden, was Schmerzen verursacht. Ausserdem dürfen die Haut – und bei oraler Verabreichung von Psoralen auch die Augen – mehrere Stunden nach Verabreichung des Medikaments nicht dem Tageslicht ausgesetzt werden, da sonst erhebliche Schädigungen auftreten können. Dies bedeutet, dass die Patienten entweder mehrere Stunden in der Klinik verweilen oder gar stationär behandelt werden müssen. Die Bestrahlungszeiten reichen je nach Hautempfindlichkeit von wenigen Minuten bis zur Grössenordnung von einer Stunde.

Diese PUVA-Therapie erfordert im Durchschnitt 20 bis 25 Bestrahlungen, wobei im Laufe der Therapie eine deutliche Hautbräunung (Pigmentierung) auftritt. Von Bedeutung ist schliesslich noch bei oraler Verabreichung von Psoralen, dass sich bei einem nicht unerheblichen Teil der Patienten eine schlechte Verträglichkeit dieses Medikaments ergibt.

Ein weiteres photochemotherapeutisches Verfahren ist durch die DE-A 2 803 446 bekannt. Dabei findet eine Bestrahlung im Wellenlängenbereich zwischen 280 und 400 nm Verwendung, wobei die Strahlung im UV-B-Bereich etwa 40% und im UV-A-Bereich etwa 60% beträgt. In Verbindung mit der Bestrahlung wird ein Präparat auf die erkrankten Hautoberflächen aufgetragen.

Im Unterschied zu diesen photochemotherapeutischen Verfahren werden bei der Phototherapie die therapeutischen Effekte der UV-Strahlung ohne zusätzlichen Einsatz von Medikamenten benutzt.

Frühere Verfahren verwendeten Quecksilberdampf-Hochdruckstrahler (Heimsonnen). Heute gibt es eine Anzahl verschiedener UV-Strahlungsquellen mit unterschiedlicher Spektralverteilung. So werden zur Therapie von Psoriasis UV-Leuchtstofflampen verwendet, die als UV-B-Lampen bezeichnet werden und deren Emissionsspektrum im UV-Bereich von etwa 270 nm bis ca. 365 nm reicht. Mehr als die Hälfte der UV-Energie wird dabei im Bereich zwischen 270 und 315 nm abgestrahlt.

Teilweise finden auch UV-Leuchtstofflampen Verwendung, deren UV-B-Anteil (unter 320 nm) bei wenigen Prozent liegt, deren UV-A-Anteil (über 320 nm) jedoch sehr hoch ist. Diese Lampen werden häufig als UV-A-Lampen («Blacklight») bezeichnet. Ihr Emissionsspektrum reicht im UV-Bereich von etwa 300 bis 400 nm.

Für die sog. SUP-Therapie (selektive UV-Therapie) werden meist Quecksilberdampf-Hochdruckstrahler mit Metallhalogendampf-Dotierungen verwendet, deren Emissionsspektrum im UV-Bereich sich von ca. 250 nm bis 400 nm erstreckt. Als therapeutisch wirksam wird hierbei der Wellenlängenbereich von ca. 290 bis 335 nm angesehen.

Bei diesem phototherapeutischen Verfahren beginnt man zum Teil mit Bestrahlungszeiten, die noch kein Erythem (Sonnenbrand) hervorrufen. Im Verlaufe der Therapie wird die Dosis jedoch so gesteigert, dass Erytheme auftreten. Das minimale Erythem ist die geringste wahrnehmbare Hautrötung; sie wird auch als Erythemschwelle bezeichnet. Je intensiver ein Erythem ist, um so unangenehmer sind seine Begleiterscheinungen. Sie entsprechen denen des Sonnenbrandes bzw. denen von Verbrennungen (Spannen, Jucken, Schmerzen sowie Rötung, Schälen, Blasenbildung, Fieber).

Ein weiteres Verfahren zur therapeutischen Behandlung der Psoriasis ist als SHIP (super hochintensive Phototherapie) bekannt. Es nützt den Wellenlängenbereich von 320 bis 330 nm aus. Die unter 320 nm liegenden Strahlungsanteile werden weitgehend ausgefiltert, nicht jedoch die Strahlung oberhalb von 330 nm. Die Bestrahlungszeiten liegen im Bereich einer halben Stunde. Bis zum Therapie-Erfolg sind im Mittel etwa 30 Bestrahlungen notwendig. Auch bei diesem Verfahren tritt als Begleiterscheinung eine Pigmentierung der Haut auf.

Zur Phototherapie der Psoriasis ist ferner ein Verfahren bekannt (DE-A 2 707 920), bei dem Hg-Niederdruckbrenner Verwendung finden, die in einem Selektionsbereich zwischen etwa 300 und 330 nm eine intensive Strahlung abgeben, wobei in den Strahlengang ein Filter geschaltet ist, dessen untere Absorptionskante bei etwa 295 nm liegt. Eine nähere Auswertung der Fig. 3 und 4 von DE-A 2 707 920 (deren Ordinatenwerte in logarithmischer Teilung aufgetragen sind) zeigt allerdings, dass bei diesem bekannten Verfahren beträchtliche Strahlungsanteile sowohl unterhalb von 300 nm als auch oberhalb von 310 nm vorhanden sind.

Ausgehend von diesem Stand der Technik liegt der Erfindung die Aufgabe zugrunde, ein UV-Bestrahlungsgerät zur Phototherapie von Dermatosen, insbesondere von Psoriasis, zu entwickeln, das es gestattet, mit kurzen Bestrahlungszeiten und einer geringen Anzahl von Bestrahlungen eine wirksame Heilung zu erzielen, ohne hierbei unerwünschte Nebenwirkungen hervorzurufen.

Diese Aufgabe wird bei dem erfindungsgemässen UV-Bestrahlungsgerät durch die kennzeichnenden Merkmale des Anspruches 1 gelöst.

Der Erfindung liegt die Erkenntnis zugrunde, dass für die Phototherapie der Psoriasis die UV-Strahlung im Wellenlängenbereich zwischen 300 und 310 nm am günstigsten ist, da sie die Zellteilungsrate (Zellproliferation) in der Epidermis verlangsamt. Die bewusste Ausnutzung dieses Wellenlängenbereiches zwischen 300 und 310 nm unterscheidet das erfindungsgemässe UV-Bestrahlungsgerät von den oben geschilderten bekannten Geräten, die im Bereich zwischen 320 und 330 nm bzw. zwischen 295 und 330 nm arbeiten. Der demgegenüber durch die Erfindung erzielte wesentliche technische und therapeutische Fortschritt wird verständlich, wenn man berücksichtigt, dass die sog. Psoriasis-Schwelle (d.h. die Bestrahlungsdosis, bei der ein Psoriasis-Therapie-Erfolg einsetzt) bereits im Wellenlängenbereich zwischen 310 und 320 nm eine Bestrahlungsdosis erfordert, die etwa um eine Zehnerpotenz über der im Wellenlängenbereich zwischen 300 und 310 nm notwendigen Bestrahlungsdosis liegt.

Auf der anderen Seite ist für die Erfindung die Erkenntnis wesentlich, dass die im Wellenlängenbereich unter 300 nm vorhandene Bestrahlungsstärke $E_2$ wesentlich kleiner als die Bestrahlungsstärke $E_1$ im Wellenlängenbereich zwischen 300 und 310 nm sein muss. Erwünscht wäre an sich eine möglichst weitgehende Unterdrückung jeglicher Strahlung im Wellenlängenbereich unter 300 nm. Dies ist jedoch im Hinblick auf die erwünschte hohe Bestrahlungsstärke im Wellenlängenbereich zwischen 300 und 310 nm mit den derzeit zur Verfügung stehenden Filtern bei vertretbarem Aufwand nicht ohne weiteres möglich (endliche Kantenneigung der Filter). Erfindungsgemäss wird daher die Bestrahlungsstärke $E_2$ im Wellenlängenbereich unter 300 nm so begrenzt, dass bei der therapeutischen Behandlung die Psoriasis-Schwelle (d.h. die Therapieschwelle) überschritten wird, noch ehe die Erythemschwelle erreicht wird.

Liegt die durch das erfindungsgemässe UV-Bestrahlungsgerät erzeugte Bestrahlungsdosis (hierunter ist das Zeitintegral der Bestrahlungsstärke zu verstehen) zwischen dem 0,7- und 1,0-fachen der Erythem-Schwellendosis, so gewährleistet dies einerseits (unter Berücksichtigung individueller Schwankungen) die zuverlässige Auslösung eines Psoriasis-Therapie-Effektes, vermeidet jedoch andererseits, dass die Erythemschwelle überschritten wird.

Damit wird bei der Verwendung des erfindungsgemässen UV-Bestrahlungsgerätes zugleich eine Pigmentierung der Haut vermieden, was im Unterschied zu den oben erläuterten bekannten phototherapeutischen Verfahren steht. Es hat sich nämlich herausgestellt, dass eine als Nebeneffekt der phototherapeutischen Behandlung eintretende Pigmentierung therapeutisch durchaus Nachteile besitzt, da das Melanin, das die Pigmentierung hervorruft, einen grossen Teil der eindringenden UV-Strahlung absorbiert, ehe sie in der Basalschicht therapeutisch wirksam werden kann. Infolgedessen muss bei Pigmentierung die Bestrahlungszeit durchweg verlängert werden. Das erfindungsgemässe UV-Bestrahlungsgerät ermöglicht demgegenüber eine wesentliche Verkürzung der Bestrahlungszeit bzw. eine Verringerung der Anzahl notwendiger Bestrahlungen.

Die erfindungsgemäss vorgesehene Begrenzung der unter 300 nm liegenden Bestrahlungsstärke ist auch unter dem weiteren Gesichtspunkt wesentlich, dass diese Strahlung (unter 300 nm) in Verbindung mit Strahlung im Wellenlängenbereich zwischen 300 und 310 nm zu einer Umkehrung des Therapieeffektes führen kann.

Gemäss einer zweckmässigen Ausgestaltung des erfindungsgemässen Bestrahlungsgerätes liegt die in der Nutzfläche vorhandene Bestrahlungsstärke $E_1$ im Wellenlängenbereich zwischen 300 und 310 nm zwischen 0,5 und 200 Wm$^{-2}$, vorzugsweise zwischen 1 und 100 Wm$^{-2}$, vorzugsweise zwischen 2 und 80 Wm$^{-2}$, vorzugsweise zwischen 5 und 50 Wm$^{-2}$.

Strahlung zwischen 800 und 1400 nm wird zweckmässig verringert, Strahlung über 1400 nm zweckmässig vollständig unterdrückt. Beides kann durch Verwendung einer 5 bis 15 mm, vorzugsweise etwa 10 mm, starken Filterschicht aus Wasser geschehen.

Strahlung zwischen 400 und 600 nm wird – vorzugsweise durch Verwendung eines Filters aus Blauviolett- oder Schwarzglas – zweckmässig weitgehend unterdrückt.

Gleiches gilt für Strahlung zwischen 330 und 440 nm, was durch Verwendung eines UV-durchlässigen Grünlichgelbglases erfolgt.

Ein erfindungsgemässes UV-Bestrahlungsgerät kann als Strahlungsquelle mindestens einen Metallhalogendampfstrahler (ohne Quecksilber) verwenden. Er besitzt den Vorteil, dass er beim Einschalten sofort den grössten Teil seiner Leistung abgibt, was vor allem bei kurzen Bestrahlungszeiten von Bedeutung ist.

Eine andere Ausgestaltung des erfindungsgemässen Bestrahlungsgerätes verwendet mindestens einen Quecksilberdampf-Hochdruckstrahler mit Metallhalogendampf-Dotierung.

Als Metallhalogene können bei beiden vorstehend genannten Strahlerarten einzeln oder in Kombination Eisen-, Nickel-, Kobalt-, Zinn-, Zink-, Indium-, Gallium-, Thallium-, Antimon und/oder Wismuthalogene verwendet werden.

Eine weitere Variante des erfindungsgemässen Bestrahlungsgerätes, die sich durch eine besonders preiswerte Herstellung auszeichnet, verwendet als Strahlungsquelle mindestens eine UV-B-Leuchtstofflampe. Sie kann anstelle von elementarem Quecksilber ein Amalgam, vorzugsweise Indium-Amalgam, enthalten und zeichnet sich dann durch eine besonders hohe Strahlungsausbeute aus.

Zur Unterdrückung der Strahlung unter 300 nm enthält das Bestrahlungsgerät zweckmässig ein Kantenfilter, dessen mittlere Transmission zwischen 300 und 310 nm mindestens gleich dem 2,8-fachen der mittleren Transmission zwischen 295 und 300 nm und mindestens gleich dem 5-fachen der mittleren Transmission zwischen 250 und 300 nm beträgt.

Das Kantenfilter kann aus organischem Material bestehen. Das organische Material kann Acrylglas Polymethylmethacrylat (PMMA) sein, in dem organische Absorber gelöst sind.

Das organische Material des Kantenfilters kann ferner Polyvinylchlorid (PVC) oder Polyäthylenterephthalat sein.

Das Kantenfilter kann ferner als Interferenzfilter ausgebildet und in einer Wasserschicht angeordnet sein.

Findet eine UV-B-Leuchtstofflampe Verwendung, so kann das Kantenfilter als Folie oder Lackschicht direkt auf die Lampe aufgebracht werden.

Eine weitere, besonders vorteilhafte Ausgestaltung des erfindungsgemässen Bestrahlungsgerätes verwendet eine UV-B-Leuchtstofflampe, deren Kolben ein farbloses, etwa 1 mm starkes Soda-Kalk-Glas besitzt und als Leuchtstoff ceraktiviertes Strontiumaluminat, bleiaktiviertes Strontiumaluminat, ceraktiviertes Strontiumfluorid und/oder ceraktiviertes Calciumfluorid verwendet. Durch diese Glas-Leuchtstoff-Kombination wird die unter 300 nm liegende Bestrahlungsstärke in dem erfindungsgemäss vorgesehenen Masse unterdrückt, so dass ein gesonderter Kantenfilter entbehrlich ist.

Zur weiteren Erläuterung der Erfindung dienen folgende Beispiele mit am Prioritätstage auf dem Markt erhältlichen Strahlungsquellen und Filtern:

Beispiel 1

Verwendet werden zwei Metallhalogendampfstrahler mit Wismuthalogen-Füllung (ohne Quecksilber) mit einer Leistung von 1000 W pro Strahler.

Der Strahlungsfluss (zwischen 300 und 310 nm) beträgt ca. 40 W pro Strahler.

Benutzt wird ein Kantenfilter des Typs WG 305 (4 mm) der Firma Schott/Mainz, ferner ein Reflektor aus eloxiertem Aluminium.

Die Bestrahlungsstärke $E_1$ (im Wellenlängenbereich zwischen 300 und 310 nm) beträgt ca. 8 $Wm^{-2}$.

Die Bestrahlungsstärke $E_2$ (im Wellenlängenbereich unter 300 nm) beträgt 0,12 $E_1$.

Die Psoriasis-Schwellenzeit $t_{s,ps}$ liegt bei 75 s und die Erythem-Schwellenzeit $t_{s,er}$ bei 90 s.

Lässt man dagegen (bei Verwendung der gleichen Strahler) den Filter weg, so ergeben sich für die Bestrahlungsstärken und die Schwellenzeiten folgende Werte:

$E_1 = 20\ Wm^{-2}$
$E_2 = 0,5\ E_1$
$t_{s,ps} = 20\ s$
$t_{s,er} = 10\ s$

Man erkennt, wie bei Verwendung einer ungefilterten Strahlung durch die vergleichsweise hohe Bestrahlungsstärke $E_2$ (im Wellenlängenbereich unter 300 nm) die Erythem-Schwelle früher als die Psoriasis-Schwelle erreicht wird.

Beispiel 2

Verwendet werden fünf Metallhalogendampf-Hochdruckstrahler mit Eisenhalogen und Quecksilber (Leistung 3000 W pro Strahler).

Der Strahlungsfluss beträgt (im Wellenlängenbereich zwischen 300 und 310 nm) ca. 80 W pro Strahler. Als Filter wird Tempax (3,4 mm) der Firma Schott/Mainz verwendet. Dieser Filter ist ebenso wie der im Beispiel 1 benutzte Kantenfilter WG 305 ein farbloses Glas mit steiler Absorptionskante im UV-Bereich. Der Reflektor besteht wieder aus eloxiertem Aluminium.

Es ergeben sich hierbei folgende Werte der Bestrahlungsstärke $E_1$ (im Wellenlängenbereich zwischen 300 und 310 nm) bzw. von $E_2$ (im Wellenlängenbereich unter 300 nm), ferner nachstehende Werte der Psoriasis-Schwellenzeit und der Erythem-Schwellenzeit:

$E_1 = 50\ Wm^{-2}$
$E_2 = 0,14 \cdot E_1$
$t_{s,ps} = 8\ s$
$t_{s,er} = 10\ s$

Lässt man dagegen den Filter weg, so ergeben sich folgende Werte:

$E_1 = 100\ Wm^{-2}$
$E_2 = 5 \cdot E_1$
$t_{s,ps} = 3\ s$
$t_{s,er} = 0,5\ s$

Beispiel 3

Verwendet man zehn UV-B-Leuchtstofflampen mit einer Leistung von 100 W pro Lampe. Der Strahlungsfluss beträgt 2 W pro Lampe (im Bereich 300–310 nm).

Als Filter wird Weich-PVC mit einer Stärke von 0,45 mm benutzt. Der Reflektor besteht aus eloxiertem Aluminium.

Hierbei ergeben sich folgende Werte:
$E_1 = 6 \ Wm^{-2}$
$E_2 = 0,12 \cdot E_1$
$t_{s,ps} = 100 \ s$
$t_{s,er} = 100 \ s$
Bei Weglassen des Filters ergeben sich folgende Werte:
$E_1 = 11 \ Wm^{-2}$
$E_2 = 0,9 \cdot E_1$
$t_{s,ps} = 30 \ s$
$t_{s,er} = 20 \ s$

Beispiel 4

Benutzt werden zwei Metallhalogendampfstrahler mit Wismuthalogen-Füllung (ohne Quecksilber) und einer Leistung von 2000 pro Strahler. Der Strahlungsfluss beträgt ca. 80 W pro Strahler.

Verwendet werden die Kantenfilter WG 305 (3 mm), GG 19 (1,5 mm) und UG 11 (3 mm) der Firma Schott/Mainz sowie eine Wasser-Filterschicht von 10 mm. Hierdurch wird auch die Strahlung oberhalb 310 nm weitgehend unterdrückt. Von den drei genannten Kantenfiltern der Firma Schott ist WG 305 ein farbloses Glas mit steiler Absorptionskante im UV-Bereich, GG 19 ein Grünlichgelbglas mit UV-Durchlässigkeit und UG 11 ein UV-durchlässiges Schwarzglas.

Der Reflektor besteht auch bei diesem Ausführungsbeispiel aus eloxiertem Aluminium.

Es ergeben sich folgende Werte:
$E_1 = 4 \ Wm^{-2}$
$E_2 = 0,04 \cdot E_1$
$t_{s,ps} = 160 \ s$
$t_{s,er} = 200 \ s$

Beispiel 5

Verwendet werden zehn UV-B-Leuchtstofflampen mit einem farblosen, etwa 1 mm starken Soda-Kalk-Glas-Kolben und einem Leuchtstoff, der durch ceraktiviertes Strontiumaluminat, bleiaktiviertes Strontiumfluorid und/oder durch ceraktiviertes Calciumfluorid gebildet wird.

Hierbei ergeben sich
$E_1 = 2,2 \ Wm^{-2}$
$E_2 = 0,19 \cdot E_1$
$t_{s,ps} = 210 \ s$
$t_{s,er} = 240 \ s$
Die vorstehenden 5 Beispiele beziehen sich auf Ganzkörper-Bestrahlungsanlagen. Werden die Beispiele 1, 2 und 4 als Teilkörper-Bestrahlungsanlagen ausgeführt, so lassen sich wegen der Verkleinerung der Nutzfläche wesentlich höhere Bestrahlungsstärken erzielen.

Beispiel 6

Für eine Teilkörper-Bestrahlung findet ein Metallhalogendampfstrahler mit Eisenjodid und Quecksilber und einer Leistung von 3000 W Verwendung, ferner ein Filter Tempax (3,4 mm) – wie im Beispiel 2 – sowie ein Filter UG 11 (3 mm) – wie im Beispiel 4 –. Gemessen wurden:
$t_{s,ps} = 11 \ s$

$t_{s,er} = 14 \ s$

Beispiel 7

Für eine Ganzkörper-Bestrahlung finden 5 Strahler wie im Beispiel 6, jedoch mit einer Leistung von 4000 W (pro Strahler) Verwendung, ferner dieselben Filter wie im Beispiel 6. Hierbei werden gemessen:
$t_{s,ps} = 33 \ s$
$t_{s,er} = 42 \ s$

Beispiel 8

Für eine Teilkörper-Bestrahlung findet derselbe Strahler wie im Beispiel 6 Verwendung. Zusätzlich zu den dort angegebenen beiden Filtern wird noch der Filter GG 19 – wie im Beispiel 4 – verwendet. Hierbei werden ermittelt:
$t_{s,ps} = 110 \ s$
$t_{s,er} = 140 \ s$

Beispiel 9

Für eine Ganzkörper-Bestrahlung finden dieselben Strahler wie im Beispiel 7 Verwendung. Zusätzlich zu den beiden dort genannten Filtern wird noch der Filter GG 19 – wie im Beispiel 4 – benutzt. Gemessen werden hierbei:
$t_{s,ps} = 330 \ s$
$t_{s,er} = 420 \ s$

Beispiel 10

Für eine Ganzkörper-Bestrahlung (unter Verwendung eines Aluminium-Reflektors) finden zwei Wismuthalogen-Strahler (ohne Quecksilber) à 1000 W Verwendung, ferner ein Filter Tempax (3,4 mm) und UG 11 (3 mm).

Hierbei ergeben sich folgende Werte:
$t_{s,ps} = 110 \ s$
$t_{s,er} = 135 \ s$

Beispiel 11

In Abwandlung des Beispieles 10 finden fünf Wismuthalogen-Strahler (mit Quecksilber) à 3000 W Verwendung, ferner ein Filter Tempax (3,4 mm). Gemessen werden hierbei:
$t_{s,ps} = 5 \ s$
$t_{s,er} = 8 \ s$

Beispiel 12

In Abwandlung von Beispiel 11 werden fünf Wismuthalogen-Strahler (mit Quecksilber) à 3000 W benutzt, ferner ein Filter Tempax (3,4 mm) und ein Filter UG 11 (3 mm). Gemessen werden
$t_{s,ps} = 8 \ s$
$t_{s,er} = 12 \ s$

Beispiel 13

In Abwandlung des Beispieles 12 werden für eine Ganzkörper-Bestrahlung fünf Wismuthalogen-Strahler (mit Quecksilber) à 3000 W benutzt, ferner die Filter Tempax (3,4 mm), UG 11 (3 mm) sowie GG 19 (1,5 mm). Hierbei werden gemessen
$t_{s,ps} = 55 \ s$
$t_{s,er} = 100 \ s$

**Patentansprüche**

1. UV-Bestrahlungsgerät zur Phototherapie von Dermatosen, insbesondere von Psoriasis, enthaltend eine im UV-B-Bereich abstrahlende Strahlungsquelle und einen die Strahlung unter 300 nm unterdrückenden Kantenfilter, dadurch gekennzeichnet, dass das Gerät eine UV-Strahlung erzeugt,

a) deren in der Nutzfläche vorhandene Bestrahlungsstärke $E_1$ im Wellenlängenbereich zwischen 300 und 310 nm zwischen 0,5 und 200 $Wm^{-2}$ liegt und

b) deren in der Nutzfläche vorhandene Bestrahlungsstärke $E_2$ im Wellenlängenbereich unter 300 nm wesentlich geringer ist als die Bestrahlungsstärke $E_1$ im Wellenlängenbereich zwischen 300 und 310 nm, wobei $E_2$ maximal 0,35 $E_1$ beträgt, wenn die Bestrahlungsstärke unterhalb einer oberen Wellenlängengrenze von 295 nm nicht grösser als 0,01 $E_1$ ist, wobei $E_2$ maximal 0,2 $E_1$ beträgt, wenn die Bestrahlungsstärke unterhalb einer oberen Wellenlängengrenze von 290 nm nicht grösser als 0,01 $E_1$ ist, wobei $E_2$ maximal zwischen 0,35 $E_1$ und 0,2 $E_1$ liegt, wenn die Bestrahlungsstärke unterhalb einer zwischen 295 nm und 290 nm liegenden Wellenlänge nicht grösser als 0,01 $E_1$ ist, und wobei in allen übrigen Fällen $E_2$ maximal 0,2 $E_1$ beträgt.

2. Bestrahlungsgerät nach Anspruch 1, dadurch gekennzeichnet, dass die in der Nutzfläche vorhandene Bestrahlungsstärke $E_1$ im Wellenlängenbereich zwischen 300 und 310 nm zwischen 1 und 100 $Wm^{-2}$, vorzugsweise zwischen 2 und 80 $Wm^{-2}$, vorzugsweise zwischen 5 und 50 $Wm^{-2}$, liegt.

3. Bestrahlungsgerät nach Anspruch 1, dadurch gekennzeichnet, dass die Strahlung zwischen 400 und 600 nm, vorzugsweise durch einen Filter aus Blauviolett- oder Schwarzglas, unterdrückt ist.

4. Bestrahlungsgerät nach Anspruch 1, dadurch gekennzeichnet, dass die Strahlung zwischen 330 und 400 nm, vorzugsweise durch ein UV-durchlässiges Grünlichgelbglas, unterdrückt ist.

5. Bestrahlungsgerät nach Anspruch 1, dadurch gekennzeichnet, dass als Strahlungsquelle mindestens ein Metallhalogendampfstrahler ohne Quecksilberzusatz vorgesehen ist.

6. Bestrahlungsgerät nach Anspruch 1, dadurch gekennzeichnet, dass als Strahlungsquelle mindestens ein Quecksilberdampf-Hochdruckstrahler mit Metallhalogendampf-Dotierung vorgesehen ist.

7. Bestrahlungsgerät nach Anspruch 5 oder 6, dadurch gekennzeichnet, dass als Metallhalogene einzeln oder in Kombinationen Eisen-, Nickel-, Kobalt-, Zinn-, Zink-, Indium-, Gallium-, Thallium-, Antimon- und Wismut-Halogene vorgesehen sind.

8. Bestrahlungsgerät nach Anspruch 1, dadurch gekennzeichnet, dass zur Unterdrückung der Strahlung unter 300 nm ein Kantenfilter vorgesehen ist, dessen mittlere Transmission zwischen 300 und 310 nm mindestens gleich dem 2,8-fachen der mittleren Transmission zwischen 295 und 300 nm und mindestens gleich dem 5-fachen der mittleren Transmission zwischen 250 und 300 nm ist.

9. Bestrahlungsgerät nach Anspruch 1, dadurch gekennzeichnet, dass als Strahlungsquelle wenigstens eine UV-B-Leuchtstofflampe vorgesehen ist, deren Leuchtstoff durch ceraktiviertes Strontiumaluminat, bleiaktiviertes Strontiumaluminat, ceraktiviertes Strontiumfluorid und/oder ceraktiviertes Calciumfluorid gebildet wird.

10. Bestrahlungsgerät nach Anspruch 8, bei dem als Strahlungsquelle wenigstens eine UV-B-Leuchtstofflampe vorgesehen ist, dadurch gekennzeichnet, dass der Kantenfilter als Folie oder Lackschicht direkt auf die UV-B-Leuchtstofflampe aufgebracht ist.

## Claims

1. UV radiation device for phototherapy of dermatoses, especially psoriasis, comprising a radiation source radiating in the UV-B wavelength range and an edge filter suppressing the radiation below 300 nm, characterised in that the device produces an UV radiation

a) the radiation intensity $E_1$ of which present in the effective area is between 0,5 and 200 $Wm^{-2}$ in the wavelength range between 300 and 310 nm and

b) the radiation intensity $E_2$ of which present in the effective area in the wavelength range below 300 nm is substantially less than the radiation intensity $E_1$ in the wavelength range between 300 and 310 nm, $E_2$ being at most 0,35 $E_1$ if the radiation intensity below an upper wavelength limit of 295 nm is not greater than 0,01 $E_1$, $E_2$ being at most 0,2 $E_1$ if the radiation intensity below an upper wavelength limit of 290 nm is not greater than 0,01 $E_1$, $E_2$ being at most between 0,35 $E_1$ and 0,2 $E_1$ if the radiation intensity below a wavelength between 295 and 290 nm is not greater than 0,01 $E_1$, in all other cases $E_2$ being at most 0,2 $E_1$.

2. Radiation device according claim 1, characterised in that the radiation intensity $E_1$ present in the effective area is between 1 and 100 $Wm^{-2}$ in the wavelength range between 300 and 310 nm, preferably between 2 and 80 $Wm^{-2}$, preferably between 5 and 50 $Wm^{-2}$.

3. Radiation device according claim 1, characterised in that the radiation between 400 and 600 nm is suppressed, preferably by a filter made from blue violet or black glass.

4. Radiation device according claim 1, characterised in that the radiation between 330 and 400 nm is suppressed, preferably by a UV permeable greenish yellow glass.

5. Radiation device according claim 1, characterised in that at least one metal halide vapour radiation device without mercury is provided as radiation source.

6. Radiation device according claim 1, characterised in that at least one mercury vapour high pressure radiation device doped with metal halide vapour is provided as radiation source.

7. Radiation device according claim 5 or 6, characterised in that halides of iron, nickel, cobalt, tin, zinc, indium, gallium, thallium, antimony and bismuth are provided individually or in combination as metal halides.

8. Radiation device according claim 1, characterised in that for suppressing the radiation below 300 nm an edge filter is provided the mean transmission of which between 300 and 310 nm is at least equal to 2.8 times the mean transmission between 295 and 300 nm and at least equal to 5 times the mean transmission between 250 and 300 nm.

9. Radiation device according claim 1, characterised in that as radiation source at least one UV-B fluorescent lamp is provided, the fluorescent substance of which is formed by cerium-activated strontium aluminate, lead-activated strontium aluminate, cerium-activated strontium fluoride and/or cerium-activated calcium fluoride.

10. Radiation device according claim 8 using at least one UV-B fluorescent lamp as radiation source, characterised in that the edge filter is applied directly to the UV-B fluorescent lamp as a foil or layer of varnish.

**Revendications**

1. Appareil à rayons ultraviolets de photothérapie de dermatoses, en particulier du psoriasis, comprenant une source d'irradiation rayonnant dans la plage des UV-B ainsi qu'un filtre à arête tendant à supprimer le rayonnement inférieur à 300 nm, caractérisé en ce qu'il produit un rayonnement UV,

a) dont l'intensité $E_1$ de l'irradiation présente dans la surface utile, dans la plage des longueurs d'onde comprise entre 300 et 310 nm, est comprise entre 0,5 et 200 $Wm^{-2}$, et

b) dont l'intensité $E_2$ de l'irradiation présente dans la surface utile, dans la plage des longueurs d'onde inférieure à 300 nm, est considérablement inférieure à l'intensité $E_1$ de l'irradiation dans la plage des longueurs d'onde comprise entre 300 et 310 nm, $E_2$ étant au maximum égale à 0,35 $E_1$ lorsque l'intensité de l'irradiation inférieure à une limite supérieure de longueur de 295 nm n'est pas supérieure à 0,01 $E_1$, $E_2$ étant au maximum égale à 0,2 $E_1$ lorsque l'intensité de l'irradiation inférieure à une limite supérieure de longueurs d'onde de 290 nm n'est pas supérieure à 0,01 $E_1$, $E_2$ étant au maximum compris entre 0,35 $E_1$ et 0,2 $E_1$ lorsque l'intensité de l'irradiation inférieure à une longueur d'onde comprise entre 295 et 290 nm n'est pas supérieure à 0,01 $E_1$ et $E_2$ étant au maximum égale à 0,2 $E_1$ dans tous les autres cas.

2. Appareil à rayons ultraviolets selon la revendication 1, caractérisé en ce que l'intensité $E_1$ de l'irradiation présente dans la surface utile, dans la plage des longueurs d'onde située entre 300 et 310 nm, est comprise entre 1 et 100 $Wm^{-2}$, de préférence entre 2 et 80 $Wm^{-2}$, et mieux encore entre 5 et 50 $Wm^{-2}$.

3. Appareil à rayons ultraviolets selon la revendication 1, caractérisé en ce que le rayonnement compris entre 400 et 600 nm est de préférence supprimé par un filtre en verre bleu-violet ou noir.

4. Appareil à rayons ultraviolets selon la revendication 1, caractérisé en ce que le rayonnement compris entre 330 et 400 nm est supprimé de préférence par un verre jaune verdâtre laissant passer les UV.

5. Appareil à rayons ultraviolets selon la revendication 1, caractérisé en ce que la source d'irradiation consiste en au moins une lampe à décharge à halogène-métal sans addition de mercure.

6. Appareil à rayons ultraviolets selon la revendication 1, caractérisé en ce que la source d'irradiation consiste en au moins une lampe à décharge à vapeur de mercure à haute pression contenant un dopant en phase vapeur par halogène-métal.

7. Appareil à rayons ultraviolets selon la revendication 5 ou la revendication 6, caractérisé en ce que les halogène-métal prévus individuellement ou en combinaison sont les suivants: halogènes-fer, nickel, cobalt, étain, zinc, indium, gallium, thallium, antimoine et/ou bismuth.

8. Appareil à rayons ultraviolets selon la revendication 1, caractérisé en ce que la suppression du rayonnement inférieur à 300 nm est produite par un filtre à arête dont la transmission moyenne entre 300 et 310 nm est au moins égale à 2,8 fois la transmission moyenne entre 295 et 300 nm et au moins égale à 5 fois la transmission moyenne entre 250 et 300 nm.

9. Appareil à rayons ultraviolets selon la revendication 1, caractérisé en ce que la source d'irradiation consiste en au moins un tube fluorescent à UV-B, dont la substance fluorescente est formée d'aluminate de strontium activé au cérium, d'aluminate de strontium activé au plomb, de fluorure de strontium activé au cérium et/ou de fluorure de calcium activé au cérium.

10. Appareil à rayons ultraviolets selon la revendication 8, dont la source d'irradiation comprend au moins un tube fluorescent à UV-B, caractérisé en ce que le filtre à arête consiste en un dépôt d'une feuille ou d'une couche de vernis directement sur le tube.